Europäisches Patentamt

European Patent Office

Office européen des brevets

(11) Publication number: **0 138 644 B1**

# EUROPEAN PATENT SPECIFICATION

(45) Date of publication of patent specification: **21.08.91**

(51) Int. Cl.⁵: **C12N 15/70**, C12N 15/16, C12N 15/62, C12P 21/00, //C12R1/19

(21) Application number: 84401709.5

(22) Date of filing: **23.08.84**

The file contains technical information submitted after the application was filed and not included in this specification

(54) **Novel plasmid vectors.**

(30) Priority: 23.08.83 JP 153467/83
30.03.84 JP 62981/84

(43) Date of publication of application:
**24.04.85 Bulletin 85/17**

(45) Publication of the grant of the patent:
**21.08.91 Bulletin 91/34**

(84) Designated Contracting States:
**CH DE FR GB LI**

(56) References cited:

**JOURNAL OF BACTERIOLOGY, vol. 145, no. 2, February 1981, Washington, DC N. MUTOH et al. "Specialized Transducing Bacteriophage Lambda Carrying the Structural Gene for a Major Outer Membrane Matrix Protein of Escherichia coli K-12" pages 1085-1090**

**T. Maniatis et al, Molecular Cloning, Cold Spring Harbor Laboratory, 1982, pp. 429-430**

(73) Proprietor: **MITSUBISHI KASEI CORPORATION**
**5-2, Marunouchi 2-chome Chiyoda-ku**

**Tokyo 100(JP)**

(72) Inventor: **Mizushima, Shoji**
**2845 Aza Kuroishi Oaza Hirabari Tenpaku-cho**
**Tenpaku-ku Nagoya-shi Aichi-ken(JP)**
Inventor: **Nakamura, Kenzo**
**41-1, Fujimigaoka, Yagoto Tenpaku-cho Showa-ku**
**Nagoya-shi Aichi-ken(JP)**
Inventor: **Nagahari, Kenji**
**13-6, Ogawa 1-chome**
**Machida-shi Tokyo(JP)**

(74) Representative: **Gutmann, Ernest**
**S.C. Ernest Gutmann - Yves Plasseraud 67, boulevard Haussmann**
**F-75008 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a plasmid vector and a host harboring the vector. More particularly, it relates to the plasmid vector suitable for use in expression of a protein and secretion of the expressed protein out of a host microbial cell.

The OmpF protein, which is one of the proteins constituting the outer membrane of Escherichia coli, is one of the proteins produced most abundantly in E. coli. The promoter and the ribosome binding region of the ompF gene are considered to effect the quite efficient functions. The expression of the ompF gene is controlled in a complex fashion. The ompB gene is known as a positive regulator gene for the ompF gene expression and the ompF gene can not be expressed in an ompB-deficient mutant. On the other hand, the expression is also controlled by the osmotic pressure in the culture medium, that is, the expression of the ompF gene is prevented in a high osmotic medium.

Vector designated as pMH621 containing the E. coli ompF signal sequence, as well as 12 amino acids of the mature ompF protein attached to the amino acids of a foreign polypeptide including a beta-galactosidase fragment is presented in "Maniatis, T. et al. (1982), Molecular Cloning, Cold Spring Harbor Laboratory, pages 429-430" as a vector said as having potential to cause the secretion of fusion proteins. The Authors stated nevertheless that it was not known whether the signal sequence would be sufficient for export.

The entire DNA sequence of the ompF gene was found by the present inventors. The OmpF protein is first synthesized in a precursor form having a signal peptide at the amino terminus. The signal peptide, which consists of 22 amino acids, is considered to play an essential role in the secretion of the OmpF protein out of the cytoplasmic membrane. Further, the OmpF protein, which is present abundantly in the outer membrane in the quite stable form, has a strong affinity with the peptidoglycan constituting the cell wall and this affinity permits the protein to be readily recovered from the cell and purified.

The present inventors have studied with a great effort the ompF gene coding for the outer membrane protein of Gram-negative bacteria including E. coli having the above-said properties while studying plasmids containing the ompF gene. Thus, these studies have led us to the present invention.

This invention provides a plasmid vector comprising a fragment which comprises the regulator region containing the ompF promoter of a Gram-negative bacterium, the region coding for the signal peptide of the OmpF protein and up to 30 nucleotides of the ompF structural gene, the fragment being inserted into a relaxed plasmid derived from E. coli. The invention also provides E. coli strain suitable for use as a host of the vector.

The invention will be described in detail with reference to the attached drawings.

In the drawings, Fig. 1 is a schematic view showing the construction of the plasmid according to the present invention, Fig. 2 is the restriction cleavage map of the AluI fragment containing the ompF promoter, Fig. 3 is the restriction cleavage map of the Sau3A-lpp fragment, and Fig. 4 shows the DNA sequence of pHF006βENo.62 coding for the signal peptide of the OmpF protein and the OmpF-β-endorphin fused protein and the amino acid sequence deduced therefrom.

The starting plasmid for use herein may be any relaxed plasmid derived from E. coli, that is, any E. coli-derived plasmid found in multiple copies per cell such as pBR322, pBR325, pACYC184 and the like, plasmid pBR322 being most preferred.

The plasmid vector of the invention is constructed by inserting the specified DNA fragment containing the ompF promoter into the starting plasmid. Illustrative examples of the DNA fragment are the following fragments (I), (II) and (III) containing the ompF promoter, which will be hereinafter described in more detail:

(I) AluI fragment;

(II) AluI-BglII fragment; and

(III) AluI-PstI (partial) fragment.

These DNA fragments may be obtainable in the following manner, for example:

λompFI, which is an ompF-transducing phage of E. coli K-12 (Journal of Bacteriology, 145, 1085-1090 (1980)), is cleaved with the restriction enzyme: (i) AluI, (ii) AluI and BglII, or (iii) AluI and PstI (partial digestion) followed by isolating the produced fragment to obtain the fragment (I), (II) or (III).

The DNA fragment containing a part of the ompF structural gene which has an appropriate length of 30 or less nucleotides may be prepared in the following manner:

EcoRI linkers are ligated to both ends of the AluI fragment obtained above. The ligated DNA fragment is inserted into the single EcoRI site of pBR322 or the like. The EcoRI site at the 5'-terminus of the DNA fragment in the plasmid is partially digested, filled in with the use of DNA polymerase and then religated. The remaining EcoRI site in the plasmid is then digested and treated with Bal31, so that a part of the ompF structural gene is removed to give a modified ompF structural gene having an appropriate length. It should be noted that nucleotides are removed from both ends of the open plasmid by the Bal31 treatment.

Alternatively, a foreign gene may be inserted into an appropriate restriction site of a plasmid containing the ompF structural gene which has a single EcoRI site followed by reducing the length of the ompF structural gene in the similar manner as above-mentioned.

For increase of the expression level and efficient secretion of the expressed protein, the ompF structural gene contained in the DNA fragment preferably comprises 20-26 nucleotides.

The fragment which comprises the regulator region containing the ompF promoter of a Gram-negative bacterium, the region coding for the signal peptide of the OmpF protein and 30 or less nucleotides of the ompF structural gene may be prepared either by chemically synthesizing the whole fragment or by chemically synthesizing a part of the fragment which is then ligated to the natural DNA corresponding to the remaining DNA sequence of the fragment by a method known to those skilled in the art. The DNA fragment for use herein may be obtainable from the natural ompF operons and the method of preparation will be apparent to those skilled in the art.

The thus obtained fragment may be inserted into a relaxed plasmid derived from E. coli in the following manner:

(1) a relaxed plasmid derived from E. coli is treated with the same restriction enzyme as used in the preparation of the fragment and the resulting plasmid DNA fragment is ligated to the fragment of the invention with the use of DNA ligase (or, the AluI fragment, which has blunt ends at both ends, can be ligated to a linearized plasmid having blunt ends);

(2) the fragment is inserted into a relaxed plasmid derived from E. coli at the restriction site cleaved with an appropriate restriction enzyme (preferably PstI site in the case of pBR322) by the so-called homopolymer-tailing method; or

(3) synthetic linker molecules having one or more recognition sites are ligated to both ends of the fragment and treated with the restriction enzyme which cleaves the linker at the recognition sites, and the resulting DNA fragment is inserted into a relaxed plasmid derived from E. coli at the sites cleaved by the same restriction enzyme as used above.

The method (3) will be described in more detail below:

Preferably, the synthetic linker molecule has EcoRI, HindIII, BamHI or SalI recognition site. EcoRI linker is most preferable.

Using the EcoRI linker, an EcoRI site may be formed at the terminus of the DNA fragment containing the ompF promoter in the following manner:

(i) AluI fragment;

The 528 bp AluI fragment is ligated to the EcoRI linker followed by digestion with EcoRI.

(ii) AluI-BglII fragment;

The 512 bp AluI-BglII fragment is treated with Klenow fragment of DNA polymerase I to form a blunt end at the BglII end and mixed with the EcoRI linker followed by digestion with EcoRI.

(iii) AluI-PstI (partial) fragment;

The 481 bp AluI-PstI (partial) fragment is treated with S1 nuclease to excise the single-stranded part formed by the digestion with PstI resulting in a blunt end, mixed with the EcoRI linker to ligate and digested with EcoRI.

(iv) shortened AluI fragment derived from the AluI fragment(i);

This fragment has blunt ends at both termini and, accordingly, the fragment can be treated in the similar manner as is the case of the AluI fragment.

The fragment having cohesive ends or blunt ends formed by the digestion with the specified restriction enzyme such as EcoRI is inserted into a relaxed plasmid derived from E. coli at the site cleaved by the same restriction enzyme as above.

The plasmid vector of the invention, which is obtained by insertion of the specified fragment containing the ompF promoter into a relaxed plasmid found in multiple copies per cell in E. coli, may be suitable for use as an expression vector. The expression vector may be constructed by inserting a heterologous gene coding for a useful protein having a physiological activity into an appropriate restriction site which is found downstream near the promoter. Such a cleavage site may be the restriction site at the 3'-terminus of the specified fragment containing the ompF promoter (BglII site or the like), the restriction site in the synthetic linker (EcoRI site or the like) when used, or an appropriate site in the relaxed plasmid which is downstream near the specified fragment.

In order to ensure the expression of the heterologous gene, transcription and translation of the heterologous gene should be under the control of the ompF promoter and, furthermore, the heterologous gene may occasionally be placed in phase in the correct reading frame by adding 2G-C base pairs or 4G-C base pairs to the fragment containing the ompF promoter in the conventional manner with the exception of the case of using the homopolymer-tailing method.

Moreover, the heterologous gene having different restriction sites at the 5'- and 3'-termini, respectively, can be used so as to insert the gene with the proper orientation into the plasmid vector of the invention.

According to the present invention, the plasmid vector thus obtained is introduced into a host strain of E. coli to produce the protein.

An example of the host will be E. coli HB101, λ1776 and the like. The host strain which can most suitably used to enhance production of the protein is any E. coli strain the protease activity of which is lost or reduced.

The protease activity is the activity of an enzyme which hydrolyzes a peptide linkage. "The protease activity is lost" herein means that at least one of the several protease activities which are found in E. coli is deleted (lost). "The protease activity is reduced" herein means that the protease activity is reduced to an extent less than the activity which a normal strain of E. coli such as E. coli HB101 has. Examples of E. coli strains the protease activity of which are lost are, for example, E. coli K-12 SM32, E. coli K-12 SM37, E. coli K-12 SM40 (Journal of Bacteriology, 148, 265-273 (1981)) or the like. A strain of E. coli the protease activity of which is reduced is E. coli N99 (Cell, 36, 43-49 (1984)) or the like.

Such E. coli host may be cultured and transformed with the plasmid vector by any conventional method.

The use of the plasmid vector containing the ompF gene, in which the fragment containing the ompF promoter comprises the gene coding for the signal peptide of the OmpF protein and a part of the structural gene of the OmpF protein downstream of the ompF promoter, provides the desired protein in the form of a stable fused protein comprising a part of the OmpF protein at the N-terminus.

The ompF promoter is such a strong promoter that the gene coding for the protein to be obtained is efficiently transcribed and translated. On the other hand, the signal peptide of the OmpF protein enhances the secretion of the expressed protein out of the cytoplasmic membrane and, therefore, facilitates the separation and purification of the protein produced.

The present invention will further be described in more detail with reference to the following non-limiting examples. Various modifications and changes can be made in the examples by those skilled in the art without departing the scope of the present invention.

EXAMPLE 1:

A DNA fragment containing the ompF gene was isolated from the DNA of λompFI, which is an ompF transducing phage of E. coli K-12 (Journal of Bacteriology, 145, 1085-1090 (1980)).

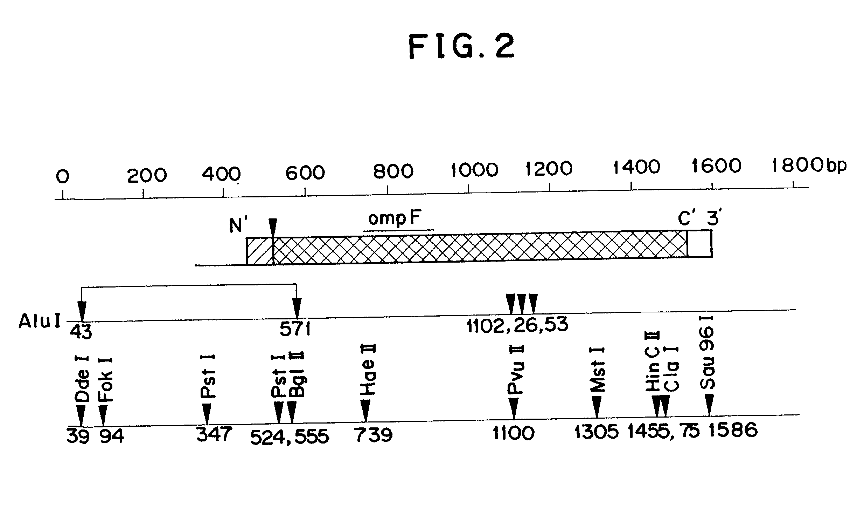

λompFI was propagated in L-broth at 30° C according to the method described by Schrenk and Weisberg in Molecular & General Genetics, 137, 101-107 (1975). The λompFI DNA was extracted with phenol in the conventional method and recovered by ethanol precipitation. The λompFI DNA was completely digested with the restriction enzyme AluI and subjected to agarose gel electrophoresis. The 528 bp AluI fragment containing the ompF promoter (Fig. 2) was eluted and purified.

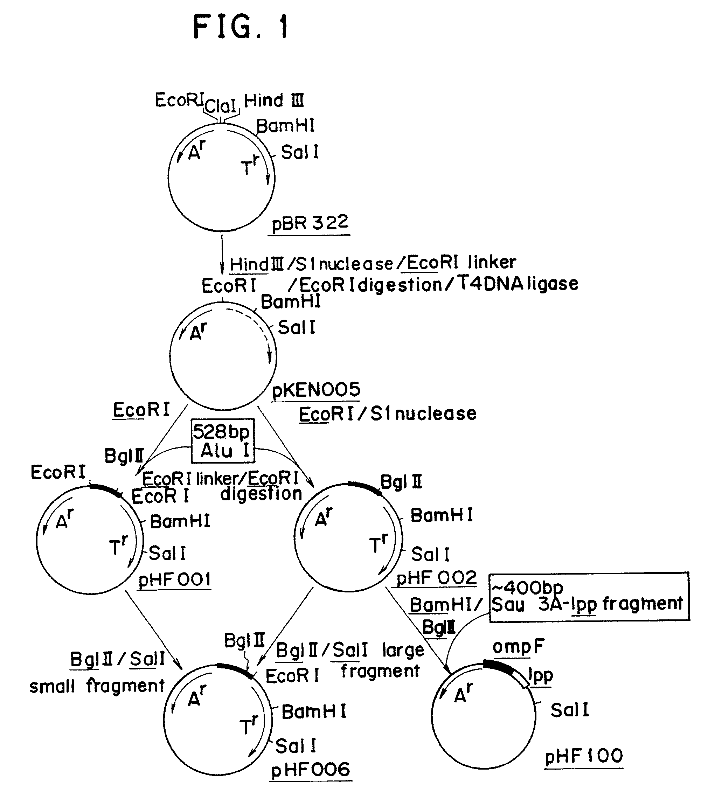

On the other hand, plasmid pBR322 was completely digested with HindIII at 37° C for one hour, treated with S1 nuclease at 20° C for one hour to remove the single-stranded portion, then treated with T4 DNA ligase at 4° C for 12 hours in the presence of EcoRI linker, digested with EcoRI at 37° C for one hour and finally treated with T4 DNA ligase to form a circular plasmid (Fig. 1). The resulting plasmid was used for transformation of E. coli HB101 and plasmid pKEN005 was obtained from the transformant.

The previously obtained AluI DNA fragment was mixed with EcoRI linker in the molar ratio of the AluI fragment to the EcoRI linker of 1 : 10, treated with T4 DNA ligase at 4° C for 12 hours and further digested with EcoRI to obtain an AluI fragment having EcoRI cohesive ends at both ends. The AluI fragment was introduced into the plasmid pKEN005 which had been digested with EcoRI at 37° C for one hour. Thus, plasmid pHF001 was obtained.

On the other hand, pKEN005 was digested with EcoRI, treated with S1 nuclease, mixed with the previously obtained AluI fragment and ligated together by blunt end ligation with T4 DNA ligase (4° C, 12 hours). The resulting plasmid was used for transformation of E. coli and plasmid pHF002 was obtained from the transformant.

The plasmid pHF002 was digested with the restriction enzymes BglII and SalI and a large fragment was obtained. The plasmid pHF001 was also digested with BglII and SalI and a small fragment was obtained. These two fragments were mixed and ligated by T4 DNA ligase at 4° C for 12 hours. The resultant plasmid was used for transformation of E. coli HB101 and plasmid pHF006 was obtained from the transformant.

Thus, the desired plasmids pHF001, pHF002 and pHF006 were obtained.

EXAMPLE 2: PRODUCTION OF LIPOPROTEIN (lpp)-LIKE PROTEIN

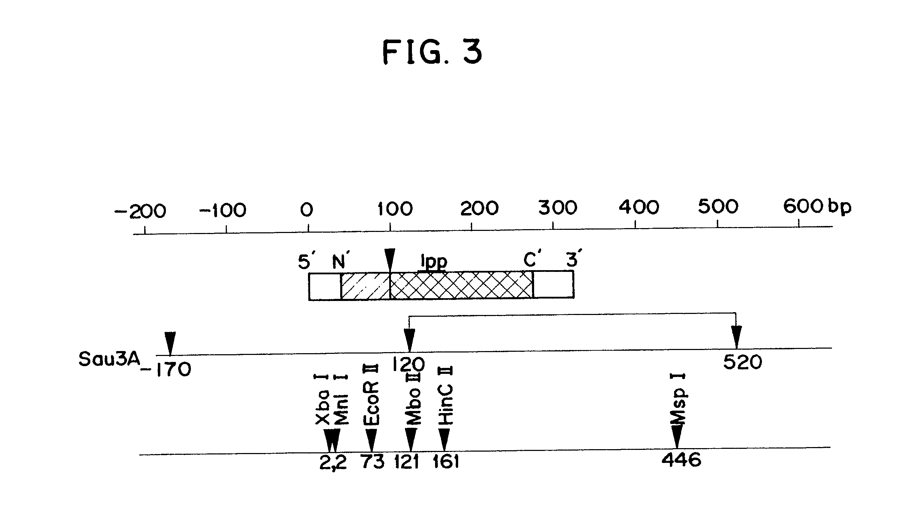

Plasmid pKEN111 (12.2 Kbp) containing E. coli lpp gene (Journal of Bacteriology, 146, 861-866 (1981)) was digested with Sau3A to give approximately 400 bp fragment containing the lpp gene a part of which at the 5'-terminus had been deleted (Fig. 3).

The BamHI-BglII fragment obtained from pHF002 by BamHI and BglII digestion was mixed with the Sau3A-lpp fragment and ligated by T4 DNA ligase at 4° C for 12 hours. The resultant plasmid was used for transformation of E. coli HB101 and plasmid pHF100 was obtained from the transformant. Then, the plasmid pHF100 was used for transformation of E. coli K-12 strain JE5513 (The Journal of Biological Chemistry, 255, 8, 3707-3712 (1980)), which is lack in lipoprotein. The resulting ampicillin resistant colony which retained pHF100 was taken for analysis.

Briefly, the bacterial membrane fraction labelled with $^{35}$S-methionine was reacted with the anti-lipoprotein antibody. The precipitated fraction was electrophoresed on SDS-polyacrylamide gel and subjected to fluorography. In the analysis, E. coli K-12 strain JE5512 (The Journal of Biological Chemistry, 255, 8, 3707-3712 (1980)) was used as lpp$^+$ strain.

The results showed that E. coli strain JE5513 retaining pHF100 reacted with the antibody, in other words, the strain produced a protein having approximately the same molecular weight as the entire lipoprotein. That is to say, a protein reacting with the antibody against lipoprotein was present in the membrane fraction of the E. coli strain JE5513 retaining pHF100.

EXAMPLE 3: PRODUCTION OF β-ENDORPHIN-LIKE PEPTIDE

I : PREPARATION OF DNA FRAGMENT CONTAINING GENE CODING FOR HUMAN β-ENDORPHIN

(A) PREPARATION OF STARTING PLASMID pA22:

Plasmid pA22, which was constructed by C. Weissmann et al., is a circular plasmid containing the amp promoter-operator region and an EcoRI site immediately after the initiation codon ATG.

Plasmid pBR322 was partially digested with MboII at 37° C for one hour, treated with S1 nuclease at 37° C for 15 minutes, then reacted with PstI linker at 15° C for 10 hours to ligate. The ligated plasmid DNA was digested with PstI at 37° C for one hour and treated with T4 DNA ligase at 15° C for 14 hours to ligate. The resulting circular plasmid was further digested with EcoRI at 37° C for one hour, treated with S1 nuclease and then treated with T4 DNA ligase at 15° C for 14 hours. The ligated plasmid was digested with PstI at 37° C for one hour, treated with S1 nuclease at 37° C for 15 minutes, reacted with EcoRI linker at 15° C for 14 hours in the presence of T4 DNA ligase and digested with EcoRI at 37° C for one hour followed by treatment with T4 DNA ligase at 15° C for 14 hours.

The resultant plasmid was used for transformation of E. coli HB101 and the plasmid DNA was prepared from the transformant according to Herenski's method (Biochemistry, 22, 4428-4440, (1970)). DNA sequencing of the plasmid was carried out by the method of Maxam and Gilbert and, consequently, the DNA sequence to be desired was confirmed.

(B) PREPARATION OF DNA FRAGMENT DERIVED FROM THE GENE FOR HUMAN CORTICOTROPIN-β-LIPOTROPIN (ACTH-β-LPH) PRECURSOR PROTEIN:

The precursor gene of approximately 11.5 Kbp was used as the starting material. The gene is considered to code for the precursor protein comprising 267 amino acid residues and may be obtained from the generally available DNA derived from human placenta or similar materials by Nakanishi et al. method (Nature, 278, 423-427 (1979)), for example.

The 11.5 Kbp fragment of the ACTH-β-LPH precursor gene was in vitro ligated in the presence of T4 DNA ligase at 15° C for 12 hours to the cleaved plasmid pBR322 which had been digested with EcoRI at 37° C for one hour.

The resulting plasmid was used for transforming E. coli χ1776. The desired transformant was selected by Grunstein-Hogness colony hybridization method (Proc. Natl. Acad. Sci. U.S.A. 72, 3961-3965 (1975)). Thus, plasmid pHLA-1 was obtained from the transformant.

The plasmid pHLA-1 was digested with EcoRI at 37° C for one hour and with BglII at 37° C for one hour. The digestion mixture was subjected to 5% polyacrylamide gel to isolate 2.0 Kbp fragment. This fragment was introduced into pBR322 instead of the 375 bp EcoRI-BamHI fragment. After transformation of E. coli, plasmid pYT1 was obtained in the same manner as described above.

The plasmid pYT1 was digested with XmaI at 37°C for one hour and 1.1 Kbp fragment containing the major portion of the ACTH-β-LPH precursor gene was isolated from 5% polyacrylamide gel. The 1.1 Kbp fragment was treated with T4 DNA polymerase at 37°C for 30 minutes to form blunt ends and then ligated with EcoRI linker at 15°C for 14 hours to give a DNA fragment of the ACTH-β-LPH precursor gene having EcoRI restriction sites near both ends.

(C) PREPARATION OF PLASMID pYT3:

The plasmid pA22 obtained in (A) and the ACTH-β-LPH fragment obtained in (B) were each digested with EcoRI at 37°C for one hour. Both digested products were ligated together at 15°C for 10 hours in the presence of T4 DNA ligase to give plasmid pYT3.

(D) PREPARATION OF DNA FRAGMENT CONTAINING GENE CODING FOR HUMAN β-ENDORPHIN:

The plasmid pYT3 was digested with HaeIII at 37°C and 160 bp fragment to be desired was isolated.

II: PREPARATION OF PLASMID pBR322trpE

Plasmid RSF2124-trp (Gene, 1, 141-152 (1977)) was digested with BglII at 37°C for one hour and the digestion mixtures were subjected to 1% agarose electrophoresis to isolate 2.3 Kbp BglII fragment. On the other hand, phage M13mp7 (7238 bp; Bethesda Research Laboratories, Inc., USA) was digested with BamHI at 37°C for one hour. This digested product was ligated to the 2.3 Kbp BglII fragment at 15°C for 14 hours with the use of T4 DNA ligase. The ligated product was used for transformation of E. coli JM103 (Bethesda Research) and the transformant was selected by the color of Xgal (5-bromo-4-chloro-3-indolyl-β-D-galactoside), that is, the color change from blue to white. RF (replicative form molecule) was obtained from the transformant and digested with EcoRI at 37°C for one hour to give EcoRI fragment, which contains the promoter, the operator, the leader region and a part of the trpE gene as well as EcoRI sites at both termini.

The EcoRI fragment was ligated at 15°C for 14 hours in the presence of T4 DNA ligase to pBR322 which had been digested with EcoRI at 37°C for one hour. The resultant plasmid was used for transforming E. coli HB101 and a plasmid containing EcoRI sites upstream and downstream of the promoter was obtained from the transformant. The resulting plasmid was partially digested with EcoRI at 37°C, treated with T4 DNA polymerase at 37°C for 30 minutes and then religated by the treatment with T4 DNA ligase at 15°C for 14 hours. The religated plasmid was used for transformation of E. coli HB101 and plasmid pBR322trpE of 6.6 Kbp was obtained from the transformant.

III: PREPARATION OF PLASMID pBR322trpEβE

The 160 bp HaeIII fragment containing the gene coding for β-endorphin, obtained in I (D), was ligated at 37°C for 14 hours in the presence of T4 DNA ligase to the fragment, which was obtained from phage M13mp7 by digestion with HincII at 37°C for one hour. After transformation of E. coli JM103, RF obtained from the transformant was digested with EcoRI at 37°C for one hour and the digestion mixtures were subjected to 5% polyacrylamide gel electrophoresis to isolate 190 bp EcoRI fragment containing the gene coding for β-endorphin.

The plasmid pBR322trpE obtained in II was digested with EcoRI at 37°C for one hour and ligated with the 190 bp fragment at 15°C for 14 hours in the presence of T4 DNA ligase. E. coli HB101 was transformed with the ligated plasmid. The transformants were analysed by mini-screening to give the desired plasmid pBR322trpEβE.

IV: PREPARATION OF PLASMID pBR322trpEβEΔλP_L

The plasmid pBR322trpEβE was digested with HpaI at 37°C for one hour in the presence of T4 DNA polymerase to protect the HpaI site in the trp promoter, further digested with SalI at 37°C for one hour and subjected to 1% agarose electrophoresis to isolate smaller fragment. On the other hand, pBR322 was digested with EcoRI at 37°C for one hour, treated with T4 DNA polymerase at 37°C for 30 minutes to form blunt end, further digested with SalI at 37°C for one hour and subjected to 1% agarose electrophoresis to isolate larger fragment. Then the small fragment and the large fragment were together treated with T4 DNA ligase at 15°C for 14 hours to ligate together. The resulting plasmid was used for transformation of E. coli HB101 and plasmid pBR322trpEβEΔλP_L was obtained from the transformant.

Then, the plasmid pBR322trpEβEΔλP_L was digested with BamHI at 37°C for one hour to obtain 171 bp BamHI fragment.

Plasmid pUC8 (Gene, 19, 259-268 (1982)) was digested with BamHI and the resulting larger BamHI fragment was ligated to the 171 bp BamHI fragment with the use of T4 DNA ligase at 4°C for 12 hours. After transformation of E. coli HB101, pUC8βE was obtained from the transformant. The plasmid was digested with EcoRI and SalI at 37°C for one hour to give 187 bp EcoRI-SalI fragment.

The plasmid pHF006, which was obtained in Example 1 (Fig. 1), was digested with EcoRI and SalI at 37°C for one hour and larger fragment was isolated. This large fragment and the 187 bp EcoRI-SalI fragment were ligated together at 4°C for 12 hours by T4 DNA ligase. After transformation of E. coli HB101, pHF006βE was obtained from the transformant.

The transformant containing pHF006βE was cultivated on M-9 medium (Experiments in Molecular Genetics, p. 431, Cold Spring Harbor Laboratory (1972)).

The grown cells were harvested at the logarithmic phase and 50 ml of the cells were subjected to fractionation to each component by the conventional methods but, in the case of the membraneous components, by the method described in Journal of Bacteriology, 145, 2, 1085-1090 (1981).

The amount of β-endorphin produced in each fraction and the medium was investigated by radioimmunoassay using $^{125}$I-β-endorphin as a label. The results are shown in Table 1.

## TABLE 1: AMOUNT OF β-ENDORPHIN

| Medium | Fractions | | | |
|---|---|---|---|---|
| | Periplasm | Cytoplasm | Inner Membrane | Outer Membrane |
| 338.4 | 0.35 | 0.25 | 0.005 | 0.14 |

(Unit: μg/l)

## PRODUCTION OF β-ENDORPHIN-LIKE PEPTIDE IN E. COLI PROTEASE ACTIVITY OF WHICH IS REDUCED

The plasmid vector pHF006βE was introduced into E. coli N99, the protease activity of which was reduced (Cell, 36, 43-49 (1984)). The resulting transformant was precultivated on M-9 medium (Experiments in Molecular Genetics, p.431, Cold Spring Harbor Laboratory (1972)) and a part of the precultured cells was inoculated on a fresh medium and further cultivated.

At each growth phase, the medium was fractionated by centrifugation and the amount of β-endorphin in the medium was determined by radioimmunoassay using $^{125}$I-β-endorphin as a label. The results are shown in Table 2.

The medium at the logarithmic phase (after 6 hours from the inoculation) was dialysed against 10 ml of ammonium bicarbonate, lyophilized to concentrate and subjected to electrophoresis on 19% SDS-polyacrylamide gel. A protein band showing β-endorphin activity in RIA was found.

TABLE 2:   AMOUNT OF β-ENDORPHIN (µg/l)

| 0* | 52.5 |
|---|---|
| 1 | 96.7 |
| 2 | 439.9 |
| 3 | 905.2 |
| 4 | 1306.1 |
| 5 | 1269.6 |
| 6 | 1269.6 |
| 7 | 1400.0 |
| 8 | 1331.3 |
| 9 | 1331.3 |
| 10 | 1243.7 |
| 11 | 1379.2 |

* Culture period after inoculation (hour)

The plasmid pHF006βE was treated with EcoRI, Bal31 and ligase by the conventional methods. The resulting DNA sample was used for transformation of E. coli N99. The plasmid purified from the amipicillin resistant transformant was digested with PstI and SalI to analyse the DNA length. Thus, the transformant harboring the plasmid from which approximately 90 base pairs in total in both directions from the EcoRI site of pHF006βE were deleted by Bal31 digestion was identified.

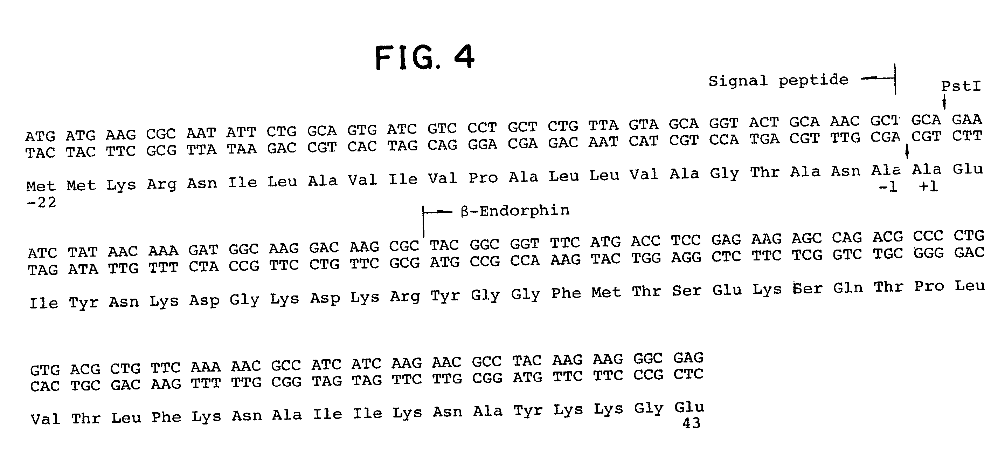

These transformant were cultured in M-9 minimal medium to the logarithmic phase and investigated the β-endorphin activity in the culture medium by radioimmunoassay to determine the transformant E. coli N99 (pHF006βENo.62) strain secreting a fused protein comprising β-endorphin into the medium. The plasmid pHF006βENo.62 was purified and DNA sequencing of the part coding for the OmpF-βE fused protein was carried out. The obtained DNA sequence as well as the deduced amino acid sequence of the fused protein to be produced are shown in Fig. 4.

The fused protein having β-endorphin activity in RIA was purified from the culture medium by concentration, desalting and HPLC. The amino acid sequencing showed that the fused protein was a protein which had the amino acid sequence deduced from the entire DNA sequence of Fig. 4 but from which the signal peptide of 22 amino acids was deleted.

**Claims**

1. A plasmid vector comprising a fragment inserted into a relaxed plasmid derived from Escherichia coli, characterized in that said fragment consists of a heterologous gene coding for a desired foreign protein downstream to a segment comprising the regulator region containing the ompF promotor of Escherichia coli, the region coding for the signal peptide of the ompF protein and 30 or less nucleotides of the ompF structural gene.

2. A plasmid vector according to claim 1, in which the segment comprises from 20 to 26 nucleotides of the ompF structural gene.

3. A method for producing a desired protein comprising introducing the plasmid vector of claim 1 or 2 into a host Escherichia coli strain, the protease activity of which is lost or reduced, cultivating the host and allowing the host cell to secrete the produced protein out of the cell.

**Revendications**

1. Vecteur plasmidique comprenant un fragment inséré dans un plasmide à l'état circulaire simple (à l'état

8

de relaxation) dérivé de Eschérichia coli, caractérisé en ce que ce fragment consiste en un gène hétérologue codant pour une protéine dérivée placé en aval d'un segment comportant une région régulatrice contenant le promoteur ompF d'Eschérichia coli, la région codant pour le peptide signal de la protéine ompF et 30 nucléotides ou moins du gène de structure ompF.

2. Vecteur plasmidique selon la revendication 1, dans lequel le segment comprend de 20 à 26 nucléotides du gène de structure ompF.

3. Procédé pour produire une protéine désirée comprenant l'introduction du vecteur plasmidique selon la revendication 1 ou 2 dans une lignée hôte de Eschérichia coli, dont l'activité protéase a été perdue ou réduite, la culture de l'hôte et la mise en condition de l'hôte cellulaire afin qu'il secrète la protéine produite hors de la cellule.

**Patentansprüche**

1. Plasmidvektor, der ein Fragment enthält, welches in ein aus Escherichia coli. stammendes entspanntes Plasmid eingefügt wurde, dadurch gekennzeichnet, daß das Fragment ein heterologes Gen enthält, das für ein gewünschtes Fremdprotein stromabwärts von einem Segment kodiert, welches die Regulationsregion des ompF Promotors von Escherichia coli., die für das Signalpeptid des ompF Proteins kodierende Region und 30 oder weniger Nukleotide des ompF Strukturgens enthält.

2. Plasmidvektor nach Anspruch 1, bei dem das Segment 20 bis 26 Nukleotide des ompF Strukturgens enthält.

3. Verfahren zur Herstellung eines gewünschten Proteins umfassend: Einschleusen des Plasmidvektors nach Anspruch 1 oder 2 in einen Escheria coli. Wirtsstamm, der keine oder verminderte Proteaseaktivität besitzt, Kultur der Wirtszellen und Veranlassen der Wirtszellen zur Sekretion des produzierten Proteins aus den Zellen.

# FIG. 1

# FIG. 2

EP 0 138 644 B1

FIG. 3

# FIG. 4

EP 0 138 644 B1

Signal peptide ──┤  PstI

```
ATG ATG AAG CGC AAT ATT CTG GCA GTG ATC GTC CCT GCT CTG TTA GTA GCA GGT ACT GCA AAC GCT GCA GAA
TAC TAC TTC GCG TTA TAA GAC CGT CAC TAG CAG GGA CGA GAC AAT CAT CGT CCA TGA CGT TTG CGA CGT CTT
```

Met Met Lys Arg Asn Ile Leu Ala Val Ile Val Pro Ala Leu Leu Val Ala Gly Thr Ala Asn Ala Ala Glu
-22                                                                              -1  +1

├── β-Endorphin

```
ATC TAT AAC AAA GAT GGC AAG GAC AAG CGC TAC GGC GGT TTC ATG ACC TCC GAG AAG AGC CAG ACG CCC CTG
TAG ATA TTG TTT CTA CCG TTC CTG TTC GCG ATG CCG CCA AAG TAC TGG AGG CTC TTC TCG GTC TGC GGG GAC
```

Ile Tyr Asn Lys Asp Gly Lys Asp Lys Arg Tyr Gly Gly Phe Met Thr Ser Glu Lys Ser Gln Thr Pro Leu

```
GTG ACG CTG TTC AAA AAC GCC ATC ATC AAG AAC GCC TAC AAG AAG GGC GAG
CAC TGC GAC AAG TTT TTG CGG TAG TAG TTC TTG CGG ATG TTC TTC CCG CTC
```

Val Thr Leu Phe Lys Asn Ala Ile Ile Lys Asn Ala Tyr Lys Lys Gly Glu
                                                                    43